# EUROPEAN PATENT SPECIFICATION

(11) **EP 2 632 950 B1**
(45) Date of publication and mention of the grant of the patent: **22.08.2018**
(21) Application number: 11720206.9
(22) Date of filing: 06.05.2011
(51) Int. Cl.: C07K 16/18, A61P 9/10, A61K 39/395

(54) **ANTIBODY BINDING TO FIBRONECTIN-EDA FOR PREVENTING MYOCARDIAL INFARCTION-RELATED COMPLICATIONS**
ANTIKÖRPER GEGEN FIBRONECTIN-EDA ZUR VERHINDERUNG VON MYOKARDINFARKTBEDINGTEN KOMPLIKATIONEN
ANTICORPS CONTRE FIBRONECTIN-EDA POUR LA PRÉVENTION DE COMPLICATIONS ASSOCIÉES À UN INFARCTUS DU MYOCARDE

(30) Priority: 26.10.2010 US 406591 P
(43) Date of publication of application: 04.09.2013
(60) Divisional application: 18182631.4
(73) Proprietor: UMC Utrecht Holding B.V., 3584 CM Utrecht (NL)
(72) Inventor: ARSLAN, Fatih, NL-3523 VV Utrecht (NL); PASTERKAMP, Gerard, 3711 BJ Austerlitz (NL); DE KLEIJN, Dominicus Paschalis Victor, NL-3962 ET Wijk Bij Duurstede (NL)
(74) Representative: V.O.
(86) International application number: PCT/NL2011/050311
(87) International publication number: WO 2012/057613

(56) References cited:
- WO-A2-2009/013619
- US-A- 4 980 279
- ARSLAN FATIH ET AL: "Lack of Fibronectin EDA Prevents Adverse Cardiac Remodeling and Preserves Cardiac Function After Acute MI", CIRCULATION, vol. 120, no. 18, Suppl. 2, November 2009 (2009-11), pages S818-S819, XP009151278, & 82ND SCIENTIFIC SESSION OF THE AMERICAN-HEART-ASSOCIATION; ORLANDO, FL, USA; NOVEMBER 14 -18, 2009 ISSN: 0009-7322
- PETERS J H ET AL: "Release of soluble fibronectin containing an extra type III domain (ED1) during acute pulmonary injury mediated by oxidants or leukocytes in vivo.", THE AMERICAN REVIEW OF RESPIRATORY DISEASE JUL 1988 LNKD- PUBMED:3202394, vol. 138, no. 1, July 1988 (1988-07), pages 167-174, XP009151378, ISSN: 0003-0805
- ARSLAN FATIH ET AL: "Lack of Fibronectin-EDA Promotes Survival and Prevents Adverse Remodeling and Heart Function Deterioration After Myocardial Infarction", CIRCULATION RESEARCH, GRUNE AND STRATTON, BALTIMORE, US, vol. 108, no. 5, 4 March 2011 (2011-03-04) , pages 582-592, XP009151261, ISSN: 0009-7330
- ZAMBROWICZ B P ET AL: "Knockouts model the 100 best-selling drugs - Will they model the next 100?", NATURE REVIEWS. DRUG DISCOVERY, NATURE PUBLISHING GROUP, GB, vol. 2, no. 1, 1 January 2003 (2003-01-01) , pages 38-51, XP002451004, ISSN: 1474-1784, DOI: 10.1038/NRD987
- CHIEN K R: "To Cre or not to Cre: the next generation of mouse models of human cardiac diseases.", CIRCULATION RESEARCH 30 MAR 2001, vol. 88, no. 6, 30 March 2001 (2001-03-30) , pages 546-549, ISSN: 1524-4571
- CRUSIO WIM E: "Flanking gene and genetic background problems in genetically manipulated mice.", BIOLOGICAL PSYCHIATRY 15 SEP 2004, vol. 56, no. 6, 15 September 2004 (2004-09-15), pages 381-385, ISSN: 0006-3223
- SELLERS R S: "The gene or not the gene--that is the question: understanding the genetically engineered mouse phenotype.", VETERINARY PATHOLOGY JAN 2012, vol. 49, no. 1, January 2012 (2012-01), pages 5-15, ISSN: 1544-2217
- FRANTZ STEFAN ET AL: "Absence of NF-kappaB subunit p50 improves heart failure after myocardial infarction.", FASEB JOURNAL : OFFICIAL PUBLICATION OF THE FEDERATION OF AMERICAN SOCIETIES FOR EXPERIMENTAL BIOLOGY SEP 2006, vol. 20, no. 11, September 2006 (2006-09), pages 1918-1920, ISSN: 1530-6860
- TIMMERS LEO ET AL: "Targeted deletion of nuclear factor kappaB p50 enhances cardiac remodeling and dysfunction following myocardial infarction.", CIRCULATION RESEARCH 13 MAR 2009, vol. 104, no. 5, 13 March 2009 (2009-03-13), pages 699-706, ISSN: 1524-4571
- SCHALKWYK L C ET AL: "Interpretation of knockout experiments: the congenic footprint.", GENES, BRAIN, AND BEHAVIOR APR 2007, vol. 6, no. 3, April 2007 (2007-04), pages 299-303, ISSN: 1601-1848
- DATABASE BIOSIS [Online] BIOSCIENCES INFORMATION SERVICE, PHILADELPHIA, PA, US 01 February 2014 SIVA KAVITHA ET AL: 'Exon-Skipping Antisense Oligonucleotides to Correct Missplicing in Neurogenetic Diseases' Database accession no. PREV201400257523 & NUCLEIC ACID THERAPEUTICS, vol. 24, no. 1, Sp. Iss. SI, 1 February 2014 (2014-02-01), pages 69-86, ISSN: 2159-3337(print), DOI: 10.1089/NAT.2013.0461
- DATABASE BIOSIS [Online] BIOSCIENCES INFORMATION SERVICE, PHILADELPHIA, PA, US February 2015 JULIER ZIAD ET AL: 'The TLR4 Agonist Fibronectin Extra Domain A is Cryptic, Exposed by Elastase-2; use in a fibrin matrix cancer vaccine' Database accession no. PREV201500273356 & SCIENTIFIC REPORTS, vol. 5, February 2015 (2015-02), page Article No.: 8569, ISSN: 2045-2322(print), DOI: 10.1038/SREP08569
- DATABASE BIOSIS [Online] BIOSCIENCES INFORMATION SERVICE, PHILADELPHIA, PA, US November 2011 MARQUES SUZANE M ET AL: 'Genetic background determines mouse strain differences in inflammatory angiogenesis' Database accession no. PREV201200022729 & MICROVASCULAR RESEARCH, vol. 82, no. 3, November 2011 (2011-11), pages 246-252, ISSN: 0026-2862, DOI: 10.1016/J.MVR.2011.08.011
- DATABASE BIOSIS [Online] BIOSCIENCES INFORMATION SERVICE, PHILADELPHIA, PA, US November 2009 VAN DEN BORNE SUSANNE W M ET AL: 'Mouse strain determines the outcome of wound healing after myocardial infarction' Database accession no. PREV200900639831 & CARDIOVASCULAR RESEARCH, vol. 84, no. 2, November 2009 (2009-11), pages 273-282, ISSN: 0008-6363(print), DOI: 10.1093/CVR/CVP207
- DATABASE BIOSIS [Online] BIOSCIENCES INFORMATION SERVICE, PHILADELPHIA, PA, US April 2008 BABAEV VLADIMIR R ET AL: 'Absence of regulated splicing of fibronectin EDA exon reduces atherosclerosis in mice' Database accession no. PREV200800410358 & ATHEROSCLEROSIS, vol. 197, no. 2, April 2008 (2008-04), pages 534-540, ISSN: 0021-9150, DOI: 10.1016/J.ATHEROSCLEROSIS.2007.08.015
- DATABASE BIOSIS [Online] BIOSCIENCES INFORMATION SERVICE, PHILADELPHIA, PA, US 01 July 2004 TAN MICHELLE H ET AL: 'Deletion of the alternatively spliced fibronectin EIIIA domain in mice reduces atherosclerosis' Database accession no. PREV200400337766 & TAN MICHELLE H ET AL: "Deletion of the alternatively spliced fibronectin EIIIA domain in mice reduces atherosclerosis", BLOOD, vol. 104, no. 1, 1 July 2004 (2004-07-01), pages 11-18, ISSN: 0006-4971
- DATABASE BIOSIS [Online] BIOSCIENCES INFORMATION SERVICE, PHILADELPHIA, PA, US 07 July 2003 MURO ANDRES F ET AL: 'Regulated splicing of the fibronectin EDA exon is essential for proper skin wound healing and normal lifespan.' Database accession no. PREV200300355658 & MURO ANDRES F ET AL: "Regulated splicing of the fibronectin EDA exon is essential for proper skin wound healing and normal lifespan.", JOURNAL OF CELL BIOLOGY, vol. 162, no. 1, 7 July 2003 (2003-07-07), pages 149-160, ISSN: 0021-9525
- DATABASE BIOSIS [Online] BIOSCIENCES INFORMATION SERVICE, PHILADELPHIA, PA, US June 2000 SIGMUND CURT D: 'Viewpoint: Are studies in genetically altered mice out of control?' Database accession no. PREV200000305578 & SIGMUND CURT D: "Viewpoint: Are studies in genetically altered mice out of control?", ARTERIOSCLEROSIS THROMBOSIS AND VASCULAR BIOLOGY, vol. 20, no. 6, June 2000 (2000-06), pages 1425-1429, ISSN: 1079-5642
- DATABASE BIOSIS [Online] BIOSCIENCES INFORMATION SERVICE, PHILADELPHIA, PA, US July 2006 SHIMAMOTO AKIRA ET AL: 'Inhibition of toll-like receptor 4 with eritoran attenuates myocardial ischemia-reperfusion injury' Database accession no. PREV200600523740 & SHIMAMOTO AKIRA ET AL: "Inhibition of toll-like receptor 4 with eritoran attenuates myocardial ischemia-reperfusion injury", CIRCULATION, vol. 114, no. Suppl. 1, July 2006 (2006-07), pages I270-I274, ISSN: 0009-7322

### SEQUENCE LISTING

<110> UMC Utrecht Holding B.V.
<120> Method for preventing myocardial infarction-related complications
<130> P30707PC00
<150> US61/406,591
   <151> 2010-10-26
<160> 16
<170> PatentIn version 3.5
<210> 1
   <211> 28
   <212> PRT
   <213> Artificial sequence
<220>
   <223> Consensus amino acid sequence corresponding to amino acids 36-60 of the EDA domain of fibronectin-EDA
<220>
   <221> misc_feature
   <222> (7)..(7)
   <223> Xaa can be Asp or Glu
<220>
   <221> misc_feature
   <222> (9)..(9)
   <223> Xaa can be Ile or Val
<220>
   <221> misc_feature
   <222> (10)..(10)
   <223> Xaa can be His or Arg or Lys
<220>
   <221> misc_feature
   <222> (13)..(13)
   <223> Xaa can be Phe or Leu or Ser
<220>
   <221> misc_feature
   <222> (17)..(17)
   <223> Xaa can be Asp or Glu
<220>
   <221> misc_feature
   <222> (19)..(22)
   <223> Xaa can be Asp or Glu
<220>
   <221> misc_feature
   <222> (27)..(27)
   <223> Xaa can be Asn or His
<400> 1
   Thr Tyr Ser Ser Pro Glu Xaa Gly Xaa Xaa Glu Leu Xaa Pro Ala Pro
   1               5                   10                  15
   Xaa Gly Xaa Xaa Xaa Xaa Thr Ala Glu Leu Xaa Gly
               20                  25
<210> 2
   <211> 27
   <212> PRT
   <213> Homo sapiens
<400> 2
   Thr Tyr Ser Ser Pro Glu Asp Gly Ile His Glu Leu Phe Pro Ala Pro
   1               5                   10                  15
   Asp Gly Glu Glu Asp Thr Ala Glu Leu Gln Gly
               20                  25
<210> 3
   <211> 21
   <212> DNA
   <213> Artificial sequence
<220>
   <223> primer collagen-1 forward
<400> 3
   tcaaggtcta ctgcaacatg g 21
<210> 4
   <211> 21
   <212> DNA
   <213> Artificial sequence
<220>
   <223> primer collagen-1 reverse
<400> 4
   aatccatcgg tcatgctctc t 21
<210> 5
   <211> 23
   <212> DNA
   <213> Artificial sequence
<220>
   <223> primer collagen-3 forward
<400> 5
   cgtaagcact ggtggacaga ttc 23
<210> 6
   <211> 22
   <212> DNA
   <213> Artificial sequence
<220>
   <223> primer collagen-3 reverse
<400> 6
   gcacatcaac gacatcttca gg 22
<210> 7
   <211> 21
   <212> DNA
   <213> Artificial sequence
<220>
   <223> primer fibronectin-EDA forward
<400> 7
   acgtggttag tgtttatgct c 21
<210> 8
   <211> 21
   <212> DNA
   <213> Artificial sequence
<220>
   <223> primer fibronectin-EDA reverse
<400> 8
   tggaatcgac atccacatca g 21
<210> 9
   <211> 20
   <212> DNA
   <213> Artificial sequence
<220>
   <223> primer p0 forward
<400> 9
   ggacccgaga agacctcctt 20
<210> 10
   <211> 24
   <212> DNA
   <213> Artificial sequence
<220>
   <223> primer p0 reverse
<400> 10
   gcacatcact cagaatttca atgg 24
<210> 11
   <211> 21
   <212> DNA
   <213> Artificial sequence
<220>
   <223> primer lysyl-oxidase forward
<400> 11
   cgcaaagagt gaagaaccaa g 21
<210> 12
   <211> 19
   <212> DNA
   <213> Artificial sequence
<220>
   <223> primer lysyl-oxidase reverse
<400> 12
   ggcatcaagc aggtcatag 19
<210> 13
   <211> 22
   <212> DNA
   <213> Artificial sequence
<220>
   <223> primer MCP-1 forward
<400> 13
   gatcggaacc aaatgagatc ag 22
<210> 14
   <211> 20
   <212> DNA
   <213> Artificial sequence
<220>
   <223> primer MCP-1 reverse
<400> 14
   gtggaaaagg tagtggatgc 20
<210> 15
   <211> 19
   <212> DNA
   <213> Artificial sequence
<220>
   <223> primer TIMP-2 forward
<400> 15
   cacccgcaac aggcgtttt 19
<210> 16
   <211> 20
   <212> DNA
   <213> Artificial sequence
<220>
   <223> primer TIMP-2 reverse
<400> 16
   tttcctccaa cgtccagcga 20

## Description

### Field of the invention

The present invention is in the field of medicine, in particular in the field of cardiology. The invention provides a method for treating, preventing, or preventing progression of myocardial infarction-related complications or conditions mediated by adverse ventricular remodeling.

### Background of the invention

Ischemic heart disease is the largest socio-economic burden to Western societies. It becomes even a bigger problem in this era of rapid modernization of developing countries like China and India. The most severe and acute complication of ischemic heart disease is a heart attack, also known as myocardial infarction. In the USA, EU and Japan only, 2.4 million patients suffer from a myocardial infarction each year¹⁻³. The amount of money spent in the USA and the EU only for the treatment of ischemic heart disease exceeds €150 billion every year^{1, 2}. Unfortunately, infarct-related complications are increasing because more patients survive the initial life-threatening infarction, but have progressively worse cardiac function hereafter. Complications after myocardial infarction (MI) such as heart failure, fibrosis and arrhythmia result in high mortality rates and morbidity. The most important determinant of these complications is an improper cardiac repair response, referred to as adverse remodeling or adverse ventricular remodeling.

Heart failure (HF) has gained much attention, as it is the most severe and most frequent consequence of adverse remodeling after myocardial infarction. It is not without reason that the largest cardiology society in the world, the European Society of Cardiology (ESC), stated "HF is the epidemic of the 21^{st} century in Western societies"⁴. In the USA, EU and Japan alone, at least 1.8 million patients are hospitalized with newly diagnosed infarct-related HF each year^{1, 2, 5}. The mortality rate is 20% within a year from diagnosis, while 50% die within 5 years⁶. Quality of life of those that survive is severely affected as they suffer from progressively decreasing exercise tolerance and reduced capacity to conduct normal daily activities⁷. The socio-economic burden is nearly €60 billion annually for the USA and EU only^{1, 2}, as a consequence of 1) the reduced exercise tolerance and subsequent reduced productivity, 2) expensive medical treatment that are not preventive but decrease symptoms and 3) hospital stays.

Current therapy for myocardial infarction aims at restoring blood flow through the occluded coronary artery. Anti-thrombotics (i.e. preventing blood clot formation) together with stents are the most important drug and device classes to optimize blood flow restoration after myocardial infarction. Despite these advances in blood flow optimization, infarction-related complications still occur and are increasing. The main reason is the fact that adverse remodeling is a completely different pathophysiological process than blood flow restoration.

The healing of the infarcted heart is a very complex process involving many types of cells⁸. Myocardial infarction is an acute event in which part of the heart muscle dies resulting in loss of pump function. Immediately after this acute event, repair processes are induced in the blood and the heart muscle characterized by enhanced inflammation. However, the type of inflammation determines whether the infarcted heart is repaired and remodeled properly. The key factor that drives improper healing and deleterious inflammation is the activation of innate immunity by molecules related to cardiac death and matrix degradation. In many patients, the immune system becomes activated in a detrimental way, resulting in inappropriate healing of the heart after myocardial infarction. In those cases, the heart will enter a process called adverse remodeling of the affected ventricle. Adverse remodeling has several deleterious consequences: heart failure, dilatation and fibrosis of the heart, disturbed contractility and relaxation, and disturbed electrical activation are known complications. The increasing incidence of infarct-related morbidity, like heart failure, emphasizes the need for novel therapeutics to enhance cardiac repair after infarction. The main determinant for leukocytes to cause a deleterious inflammatory reaction is the deposition of fibronectin-EDA. After myocardial infarction, fibronectin-EDA is newly synthesized and transiently upregulated in the infarcted myocardium. Fibronectin-EDA can activate the immune system, thereby inducing the migration of leukocytes to the infarcted heart. Subsequently, leukocytes activated by fibronectin-EDA induce detrimental inflammatory reactions in the healing heart (Arslan. F. et al. Circ. Res., Mar 2011; 108: 582 - 592).

Cellular fibronectin is a multifunctional adhesive glycoprotein present in the ECM and is produced by cells in response to tissue injury as occurs with MI. It contains an alternatively spliced exon encoding type III repeat extra domain A (EIIIA; EDA), that act as an endogenous ligand for both TLR2 and TLR4. Shimamoto et al. has reported that the inhibition of TLR4 with eritoran significantly reduces myocardial ischemia-reperfusion injury (Shimamoto et al., Circulation, 2008; 114:270-274). *In vitro,* fibronectin-EDA induces pro-inflammatory gene expression and activates monocytes. *In vivo* injection of fibronectin-EDA in murine joints results in enhanced inflammation. Thus, fibronectin-EDA is capable of activating leukocytes and cause an upregulation of cytokines and chemokines. It was recently shown that fibronectin-EDA knockout mice exhibited reduced fibrosis, preserved cardiac function and reduced ventricular dilatation compared to wild-type mice after infarction (Arslan F. et al. Circ. Res., Mar 2011; 108: 582 - 592). It was also recently demonstrated that adverse cardiac remodeling was prevented in fibronectin-EDA knockout mice as compared to wild-type mice after myocardial infarction (Arslan R. et al. Circulation, November 2009; 120: S818-S819).

In the field, improved interventions for preventing MI-related complications such as heart failure are sought for.

### Summary of the invention

The present inventors have now demonstrated that treatment of mice with antibodies directed to the EDA domain of fibronectin-EDA prevents left ventricular dilatation in said mice and improves survival after MI.

Thus, the present invention provides for an antibody that specifically binds to the amino acid sequence TYSSPEDGIHELFPAPDGEEDTAELQG (SEQ ID NO:2) of the EDA domain of fibronectin-EDA for treating, preventing, or preventing progression of a condition related to or resulting from a myocardial infarction and/or adverse cardiac remodeling. Such condition is selected from the group consisting of cardiac dilatation, adverse ventricular remodeling, heart failure; remote myocardial fibrosis; aneurysm or rupture of the ventricle; mitral regurgitation, particularly if the infarction is large and likely to cause severe ventricular dilatation; and arrhythmias, such as ventricular fibrillation and ventricular tachycardia due to ventricular enlargement and fibrosis. Preferably, the condition is selected from the group consisting of heart failure and remote myocardial fibrosis. Said heart failure may occur after myocardial infarction.

The antibody may further be used for preventing fibrosis and/or for improving cardiac function.

The antibody is preferably a neutralizing antibody, more preferably a monoclonal antibody, such as a human monoclonal antibody or a humanized monoclonal antibody, for example a humanized mouse monoclonal antibody.

### Detailed description of the invention

The present inventors have demonstrated that treatment of mice with mouse monoclonal antibodies of IgG1 and IgG2a subclass recognizing the antigen TYSSPEDGIHELFPAPDGEEDTAELQG (SEQ ID NO:2) located in the EDA domain of fibronectin-EDA prevents left ventricular dilatation in said mice and improves survival after MI. It was also found that adverse cardiac remodeling in subjects suffering from acute MI could be treated or prevented. As used herein, the term "adverse cardiac remodeling", "adverse remodeling", and "adverse ventricular remodeling" are used interchangeably.

Thus, the present invention provides for an antibody that specifically binds to the amino acid sequence TYSSPEDGIHELFPAPDGEEDTAELQG (SEQ ID NO:2) of the EDA domain of fibronectin-EDA treating, preventing, or preventing progression of a condition related to or resulting from myocardial infarction (MI) and/or adverse ventricular remodeling. Such condition is selected from the group consisting of heart failure; remote myocardial fibrosis; aneurysm or rupture of the ventricle; mitral regurgitation, particularly if the infarction is large and likely to cause severe ventricular dilatation; and arrhythmias, such as ventricular fibrillation and ventricular tachycardia due to ventricular enlargement and fibrosis. Preferably, the condition is selected from the group consisting of heart failure and remote myocardial fibrosis.

The term "treating, preventing, or preventing progression of" as used herein not only encompasses the onset of adverse cardiac remodeling, but also encompasses the situation in which adverse cardiac remodeling has commenced but is halted from continuing further. Thus, it encompasses the situation in which full-blown development of MI-related complications is prevented, even if such MI-related complications have already started to develop. For example, cardiac dilatation that has already commenced can be stopped by the therapeutic intervention using the antibody of the present invention. Such method is encompassed by the present invention. As adverse remodeling is reversible, adverse remodeling-related complications, herein also referred to as "MI-related complications", can also be treated using the method of the present invention.

As used herein, the terms "heart failure" refers to any condition characterized by decreased cardiac output and/or abnormal filling pressures in the ventricles. In these situations, the heart is unable to pump blood at an adequate rate or in adequate volume and/or in adequate force (i.e. systolic heart failure) or exhibits increased ventricular stiffness (i.e. diastolic heart failure), respectively. In heart failure, blood perfusion of organs is hampered thereby deteriorating organ function (e.g. kidney or liver failure). In addition, blood can back up into the lungs, causing the lungs to become congested with fluid. Typical symptoms of heart failure include shortness of breath (dyspnea), fatigue, weakness, difficulty breathing when lying flat, and swelling of the legs, ankles or abdomen (edema).

The term "antibody" used herein refers to any immunoglobulin or fragment thereof, and encompasses any polypeptide comprising an antigen-binding site with at least one complementarity determining region (CDR). The term includes, but is not limited to, polyclonal, monoclonal, monospecific, polyspecific, non-specific, humanized, chimeric, human, single-chain, synthetic, recombinant, hybrid, mutated, grafted and *in vitro* generated antibodies. The term "antibody" also includes antibody fragments such Fab, F(ab')₂, Fv, scFv, Fd, dAb, and other antibody fragments or other constructs comprising CDRs that retain antigen-binding function. Typicallly, such fragments would comprise an antigen-binding domain. The details of the preparation of such antibodies and their suitability for use as binding members, particularly a specific binding member, are well known to those skilled in the art. The term "neutralizing" refers to the ability of an antibody to inhibit (i.e., eliminate or reduce) at least one activity of another compound or molecule. The antibody or fragment thereof may be any of the known antibody isotypes and their conformations , for example, IgA, such as IgA1 or IgA2, IgD, IgE, IgG, such as IgG1, IgG2a, IgG2b, IgG3, IgG4, or IgM class, or may constitute mixtures thereof in any combination, such as a mixture of antibodies from the IgG1 and IgG2a class.

"Specific binding" of the antibody to fibronectin-EDA means that fibronectin-EDA and the antibody form a complex that is relatively stable under physiological conditions. Specific binding is characterized by a high affinity and a low to moderate capacity as distinguished from non-specific binding which usually has a low affinity with a moderate to high capacity. Typically, binding is considered specific when the affinity constant Kₐ is higher than 10⁶ M⁻¹. If necessary, non-specific binding can be reduced without substantially affecting specific binding by varying the binding conditions in an *in vitro* test. In the present invention an "antibody for fibronectin-EDA" or "an antibody specifically binding to fibronectin-EDA" is an antibodyin which is the only target that is bound with high affinity, i.e. other polypeptides such as fibronectin-EDB or plasma fibronectin are not bound by said specific antibody or bound only with low affinity (non-specific binding).

The antibody may be used for preventing adverse cardiac remodeling, cardiac dilatation, and/or for improving cardiac function. Said heart failure may occur after myocardial infarction.

Said antibody binds an amino acid sequence as depicted by SEQ ID NO:2. SEQ ID NO:1 represent a consensus amino acid sequence of amino acids 36 to 60 of the EDA domain of fibronectin-EDA derived from the alignment of the EDA domain of fibronectin-EDA from various animals. SEQ ID NO:2 corresponds to amino acids 36 to 60 of the EDA domain of human fibronectin-EDA.

The antibody is preferably a neutralizing antibody, more preferably a monoclonal antibody, such as a human monoclonal antibody or a humanized monoclonal antibody, for example a humanized mouse monoclonal antibody.

The antibody, may be administered to a subject in need thereof for treating, preventing, and/or preventing progression of adverse cardiac remodeling and related complications (i.e., conditions related to, or resulting from MI) as indicated above. The subject may be an animal, such as a non-human animal. Suitable subjects include, without limitation, mammals, such as humans.

The antibody may be administered in an effective amount. As used herein, the term "effective amount" refers to a quantity sufficient to achieve a desired therapeutic and/or prophylactic effect, e.g., an amount which results in the treatment, prevention of, or prevention of progression of, a condition related to or resulting from myocardial infarction (MI) and/or adverse cardiac remodeling, such as heart failure or one or more symptoms associated with heart failure. In the context of therapeutic or prophylactic applications, the amount of antibody administered to the subject will depend on the type and severity of the disease or condition and on the characteristics of the subject, such as general health, age, sex, body weight and tolerance to drugs. It will also depend on the degree, severity and type of condition. The skilled artisan will be able to determine appropriate dosages depending on these and other factors. The antibody can also be administered in combination with one or more additional therapeutic compounds. In the methods of the present invention, the antibody may be administered to a subject having one or more signs or symptoms of MI and/or heart failure, such as chest pain, dyspnea, edema and cardiomegaly. For example, a "therapeutically effective amount" of the antibody is meant levels in which the physiological effects of a condition related to or resulting from MI and/or adverse cardiac remodeling, such as heart failure are, at a minimum, ameliorated. The skilled person will be capable of determining when such condition has been treated, prevented, or when its progression has been prevented.

An effective amount of the antibody, for example, monoclonal antibody, may be in the range of about 0.1 µg/kg to about 10 g/kg, such as about 1 µg/kg to about 1 g/kg, about 10 µg/kg to about 100 mg/kg, or about 0.1 mg/kg to about 50 mg/kg.

In a suitable embodiment, the antibody may be administered intravenously, for example, by means of a bolus injection. The antibody may be administered once in a single dosage, or may be administered several times after MI. For example, the antibody may be administered intravenously, once immediately after the MI (i.e., within 48 hours of the MI), followed by one or more intravenous administrations on the days following the first administration of the antibody of the invention. The antibody may be administered with an interval ranging from about 2 hours to about 14 days, such as about 4 hours to about 10 days, about 6 hours to about 8 days, about 8 hours to about 6 days, about 12 hours to about 4 days, or about 24 hours to about 2 days to achieve optimal therapeutic or preventive effect.

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a composition of the invention may comprise additional component(s) than the ones specifically identified, said additional component(s) not altering the unique characteristics of the invention.

In addition, reference to an element by the indefinite article "a" or "an" does not exclude the possibility that more than one of the element is present, unless the context clearly requires that there be one and only one of the elements. The indefinite article "a" or "an" thus usually means "at least one".

The term "about" may, where applicable, indicate a deviation of 10% or less, or 5% or less, or 1% or less, or 0.5% or less, or even 01.% or less, and also in an embodiment no (measureable) deviation. As will be clear to the person skilled in the art, small deviations from numerical values may, where applicable, in general be allowed. Hence, except for the values in the definition of about above, numerical values may, where applicable deviate a 10% or less, or 5% or less, or 1% or less, or 0.5% or less, or even 0.1% or less from the given value. To stress this, herein sometimes the word "about" is used before numerical values.

It will be clear that the above description and the figures are included to illustrate some embodiments of the invention, and not to limit the scope of protection. Hereinafter the invention will be illustrated further by means of non-limiting examples.

### Figures

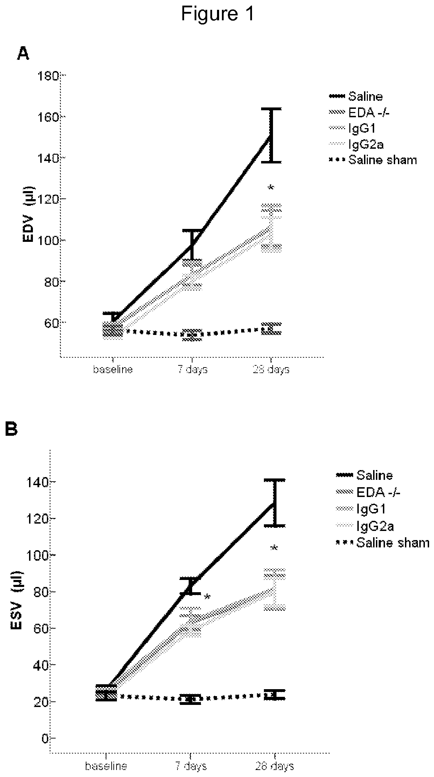

Figure 1A-D show the end-diastolic volume (A; EDV), end-systolic volume (B; ESV), systolic wall thickening (C; SWT) and Kaplan-Meier cumulative survival curve (D) after MI, in saline treated mice EDA knock-out mice (EDA^{-/-}), IgG1 anti-fibronectin-EDA antibody and IgG2a anti-fibronectin-EDA antibody treated and sham operated animals. Mice were injected with either saline or antibodies (lgG1 or IgG2a) against fibronectin-EDA 1 day after MI. Sham operated animals did not receive any treatment other than a sham operation. EDA^{-/-} mice were subjected to MI but did not receive any therapeutic intervention.

### Sequences

SEQ ID NO:1:
   TYSSPE(D/E)G(I/V)(H/R/K)EL(F/L/S)PAP(D/E)G(E/D)(E/D)(E/D)(D/E)TAEL(Q/H)G
SEQ ID NO:2:
   TYSSPEDGIHELFPAPDGEEDTAELQG

### Examples

### Example 1

### Methods

### Mice

Fibronectin-EDA knock out mice were generated as described previously by Tan et al. (Blood. 2004;104:11-18). EDA-/- mice were backcrossed into a Balb/C background for 8 generations.

### Protein and RNA isolation

Total RNA and protein was isolated from snap frozen infarcted heart sections (infarct and remote area separated) using 1 ml TripureTM Isolation Reagent (Roche, Woerden, the Netherlands) according to the manufacturers' protocol. A 40 mM Tris solution (pH 7.5) was used for protein isolation from samples harvested 7 days after infarction for zymography.

### Flow Cytometry

Tumor necrosis factor (TNF)-α, RANTES, IL10, MCP-1 and granulocyte macrophage-colony stimulating factor (GM-CSF) levels in isolated tissue protein samples were measured by flow cytometry (Cytomics FC500, Beckman Coulter) using the Th1/Th2 customized multiplex kit (Bender MedSystems, Vienna, Austria). The protein samples were diluted 1:1 in assay buffer, and the protocol was further followed according to the manufacturer's instructions. TLR2, TLR4, CD49d expression was assessed on circulating monocytes of EDTA anticoagulated blood by flow cytometry. Whole blood was stained for TLR2 (FITC, eBioscience, San Diego, Calif), TLR4 (PE, eBioscience, San Diego, Calif), CD49d (Alexa Fluor 488, Serotec, Oxford, UK) and F4/80 for monocytes (Alexa Fluor 647, Serotec, Oxford, UK).

### Generation of chimeric mice

We generated chimeric mice to study the relative contribution of EDA expression in blood and parenchymal cells to LV remodeling. Donor bone marrow (BM) cells were collected from WT and EDA-/- mice by flushing humerus, femurs and tibiae with RPMI-1640 medium. Recipient mice received 5x106 BM cells after receiving a single dose of 10 Gy to irradiate host bone marrow. Mice recovered for 6 weeks to ensure stable engraftment of the donor bone marrow cells. Hereafter, DNA was extracted from peripheral blood samples and used for genotyping with quantitative polymerase chain reaction (qPCR). Successful chimerization (>95% circulating donor cells) was achieved in all mice (data not shown). Irradiated WT mice with EDA-/- bone marrow are referred as WT/EDA KO BM, and EDA-/- mice with WT bone marrow as EDA KO/WT BM. WT/WT BM and EDA KO/EDA KO BM mice served as appropriate controls for functional and statistical comparison.

### Myocardial infarction in vivo

Mice were anesthetized with a mixture of Fentanyl (Jansen-Cilag) 0.05 mg/kg, Dormicum (Roche) 5 mg/kg and medetomidine 0.5 mg/kg through an intraperitoneal injection. Core body temperature was maintained around 37°C during surgery by continuous monitoring with a rectal thermometer and automatic heating blanket. Mice were intubated and ventilated (Harvard Apparatus Inc.) with 100% oxygen. The left coronary artery (LCA) was permanently ligated using an 8-0 vicryl suture. Ischemia was confirmed by bleaching of the myocardium and ventricular tachyarrhythmia. In sham operated animals the suture was placed beneath the LCA without ligating. The chest wall was closed and the animals received subcutaneously Antisedan (Pfizer) 2.5 mg/kg, Anexate (Roche) 0.5 mg/kg and Temgesic (Schering-Plough) 0.1 mg/kg.

### Infarct size

Infarct size (IS) as a percentage of the left ventricle (LV) was determined using Evans' blue dye injection and TTC staining, 2 days after infarction (n=6/group). By assessing infarct size in the acute phase (at 2 days), one can determine whether differences are present between WT and EDA-/- mice in myocardial perfusion. Hence, 4% Evans blue dye was injected via the thoracic aorta in a retrograde fashion. By doing so, one can demarcate the area-at-risk (AAR), the extent of myocardial tissue that underwent ischemia (i.e. endangered myocardium). Hearts were rapidly explanted, rinsed in 0.9% saline and put in -20ºC freezer for 1 hour. Hereafter, hearts were mechanically sliced into four 1-mm cross sections. Heart sections were incubated in 1% triphenyltetrazolium-chloride (Sigma-Aldrich) at 37ºC for 15 minutes before placing them in formaldehyde for another 15 minutes. Viable tissue stains red
and infarcted tissue appears white. Heart sections were digitally photographed (Canon EOS 400D) under a microscope (Carl Zeiss®). IS, AAR and total LV area were measured using ImageJ software (version 1.34). Infarct size was corrected for the weight of the corresponding heart slice. After 28 days, IS/LV was determined using hematoxylin-eosin stained cross sections.

### Magnetic resonance imaging

Fourty-two mice without bone marrow transplantation (n=15/group in ischemic and n=6/group in sham operated mice) and 44 mice (n=11/group) in the chimeric mouse experiments underwent serial assessment of cardiac dimensions and function by high resolution magnetic resonance imaging (MRI, 9.4 T, Bruker, Rheinstetten, Germany) under isoflurane anesthesia before, 7 and 28 days after MI. Long axis and short axis images with 1.0 mm interval between the slices were obtained and used to compute end-diastolic volume (EDV, largest volume) and end-systolic volume (ESV, smallest volume). The ejection fraction (EF) was calculated as 100*(EDV-ESV)/EDV. Wall thickness (WT) and systolic wall thickening (SWT) were assessed from both the septum (remote myocardium) and free wall (infarct area). All MRI data are analyzed using Qmass digital imaging software (Medis, Leiden, The Netherlands).

### LV pressure measurements

In a subset of mice, invasive assessment of cardiac performance and LV pressure development was performed 28 days after infarction. A Millar 1.4F pressure catheter (model SPR-839) was inserted in a retrograde fashion via the right common carotid artery. Systolic function was assessed by dP/dtmax, whereas diastolic function by LV end-diastolic pressure and tau (time constant of LV relaxation). Tau was determined from the regression of dP/dt versus LV pressure.

### Immunohistochemistrv

Upon termination, hearts were excised and fixated in 4% formaldehyde and embedded in paraffin. Paraffin sections were stained for Ly-6G (for neutrophils; rat anti-mouse Ly-6G 1:100, Abcam, Cambridge, United Kingdom), MAC-3 (for macrophages; rat anti-mouse MAC-3 1:30, BD Pharmingen, Breda, the Netherlands), α-SMA (for myofibroblasts; rabbit anti-human α-SMA 1:50, Acris antibodies, Herford, Germany), periostin (maturation factor; rabbit-anti human periostin 1:50, Sigma, Zwijndrecht, the Netherlands) and fibronectin-EDA (mouse anti-human 1:150, Abcam, Cambridge, United Kingdom). Quantification of collagen density was performed using Picrosirius Red staining of 4% formalin fixated and paraffin embedded heart sections. Sections were stained by overnight incubation with the first antibody at 4°C for MAC-3 and periostin or by 1 hour incubation at RT for Ly-6G and ½ hour incubation at RT
for α-SMA. Before staining, sections were deparaffinized and endogenous peroxidase was blocked by 30 minutes incubation in methanol containing 1.5% H2O2. Antigen retrieval was performed by 20 minutes boiling in citrate buffer (MAC-3, Ly-6G and periostin), 10 min boiling in citrate buffer for fibronectin-EDA or 15 minutes at 37°C in pepsin buffer (-SMA). For MAC-3, α-SMA and periostin staining, sections were pre-incubated with normal goat serum and incubated with the primary antibody (MAC-3, 1:30 overnight at 4°C; α-SMA, 1:50 for 30 minutes at RT; periostin, 1:50 overnight at 4°C). Sections were then incubated for 1 hour at RT with a biotin labeled secondary antibody, followed by 1 hour incubation with streptavidin-horseradish peroxidase at RT and developed with AEC. For Ly-6G, sections were incubated with the primary antibody (1:100 for 1 hour at RT). Sections were then incubated for 30 minutes with a secondary antibody followed by 30 minutes incubation with Powervision poly-HRP anti-rabbit IgG (ImmunoVision Technologies, Daily City, USA). The staining was immediately visualized with Vector NovaREDTM substrate kit following the manufacturer's instructions (Vector Laboratories Inc., Burlingame, USA). For Fibronectin-EDA, sections were pre-incubated with bovine serum albumin and incubated with the primary antibody (1:150 overnight at 4°C). Next day, sections were incubated with a secondary antibody (rabbit anti mouse, Dako, Glostrup, Denmark) followed by incubation for 1h with AB complex (Vector Laboratories Inc., Burlingame, USA). The staining was immediately visualized with DAB (Dako, Glostrup, Denmark). All sections were counterstained with Mayer's hematoxylin stain.

Collagen density analysis was done with circularly polarized light after conversion into grey values and digital image microscopy. Histograms were generated of the entire image, in which the number of pixels with a certain grey value was represented. Grey values below 30 were considered as background signal of the image. Myofibroblasts influx and periostin expression were calculated as the positive α-SMA and periostin area fraction. Vessels were excluded from the analysis.

### Polymerase chain reaction

RNA was isolated using Tripure reagent according to manufacturer's protocol (Roche). After DNase treatment, 500 g total RNA was used for cDNA synthesis using the iScriptTM cDNA synthesis kit (Bio-Rad). Amplification was performed using 10 µl iQTM SYBR Green supermix and 10 µl cDNA. Quantities are determined by comparison with known quantities of cloned PCR products. All mRNA expression levels were corrected for the amount of p0. Primers were designed using Beacon Designer 4.0 (Premier Biosoft): collagen-1 (forward: 5'- tcaaggtctactgcaacatgg-3'; reverse: 5'-aatccatcggtcatgctctct-3'), collagen-3 (forward: 5'-cgtaagcactggtggacagattc-3'; reverse: 5'-gcacatcaacgacatcttcagg -3'), EDA (forward: 5'-acgtggttagtgtttatgctc-3'; reverse: 5'-tggaatcgacatccacatcag-3') and p0 (forward: 5'-ggacccgagaagacctcctt-3'; reverse 5'-gcacatcactcagaatttcaatgg-3'), lysyl-oxidase (forward: 5'-cgcaaagagtgaagaaccaag-3; reverse': 5'-ggcatcaagcaggtcatag-3'), MCP-1 (forward: 5'-gatcggaaccaaatgagatcag-3'; reverse: 5'-gtggaaaaggtagtggatgc-3'), TIMP-2 (forward: 5'-cacccgcaacaggcgtttt-3'; reverse: 5'-tttcctccaacgtccagcga-3').

### Zymography

Tris isolated protein samples (5 µg) were separated on a sodium dodecyl sulfate-polyacrylamide gel containing 1 mg/ml gelatin (Sigma) in the 8% running gel. After running, the gel was washed 2x15 min in 2.5% Triton X-100 and incubated overnight at 37°C in Brij solution (0.05 M Tris-HCI pH 7.4, 0.01 M CaCI2, 0.05% Brij 35 (Sigma)). The gel was then stained with Coomassie blue (25% methanol, 15% acetic acid, 0.1% Coomassie blue) for 1 h at room temperature (RT), followed by a destaining in 25% methanol/15% acetic acid for approximately 30 min. Active MMP-2 and -9 were identified by size and in co-migration with its recombinant protein.

### Elastase activity

Elastase activity was measured in 100 µg Tris-protein extracts using EnzCheck Elastase Assay kit (E12056, Invitrogen, Breda, Netherlands) according to the manufacturer's instructions. Aliquots of the DQ elastin substrate (with a final concentration of 25 µg/ml) were added to the samples and incubated for 1-3 hours at 37 °C. Background fluorescence from a no-enzyme control reaction had been subtracted from each value.

### Myofibroblast culture

Hearts from WT and EDA-/- mice were flushed with saline and explanted 5 days after infarction. Right ventricle, atria and valves were removed. Hearts were cut into submillimeter pieces and treated for 1 hour with collagenase (2 mg/mL, Roche) at 37 C. Single cell suspensions were obtained through cell strainers. Myofibroblasts were selected based on their ability to proliferate in vitro. Myofibroblasts were expanded using DMEM culture medium (Gibco), supplemented with 10% FCS (HyClone, Logan, Utah, VS) and 1% penicillin/streptomycin (Sigma). For MMP expression profile, myofibroblast were cultured overnight in 0.1% FCS to minimize background MMP2 and -9 activitity on zymography. Immunocytochemistry was performed on cells at passage 3, seeded on coverslips and cultured for 3 days, fixed in 4%. For the staining, coverslips were washed, permeabilized with 0.1% Triton-X100 and blocked in a 2% BSA (Roche), 0.1% saponin solution in PBS, and incubated for 1 hr with primary antibodies in PBS. Antibodies used: αSMA (1:400, Sigma, A2547, stock concentration (sc): 4.5 mg/mL), vimentin (1:400, ab20346, VI-10, Abcam, sc: 1.0 mg/mL), desmin (1:50, MA1-46394,ThermoScientific) and procollagen type III (1:50, BP8034, Acris, sc: 1.0 mg/mL). Control stainings were performed by omitting primary antibody. The used secondary antibodies were: AlexaFluor-555-labeled goat-anti-rabbit (1:400, invitrogen, A21429, sc: 2mg/mL) and AlexaFlour-488-labeled goat-anti-mouse (1:400, invitrogen, A11001, 2mg/mL). Cell nuclei were stained in 1ng/mL Hoechst dye for 5 minutes. Coverslips were mounted with a 10% mowiol-solution (w/v) (25% glycerol, 50% Tris-HCI, pH 8.5). Human adult cardiomyocytes were added later as a positive control to confirm negative desminstaining. Cells were viewed by fluorescence microscopy (Olympus, BX60).

### Results

### Lack of EDA promotes survival and prevents heart function deterioration as well as maladaptive remodeling after myocardial infarction

EDA synthesis is stimulated after infarction in both infarct and remote myocardium, reaching a peak at 7 and 3 days, respectively. Baseline MRI assessment of cardiac function and dimensions revealed no differences between EDA-/- and WT mice. Microscopic analyses did not show any alterations in cellularity and matrix composition in EDA-/- mice. The extent of endangered myocardium (AAR/LV) determined at 2 days post-MI was similar between the groups. Infarct size (IS) as percentage of LV was also similar between groups (IS/LV 38.2±1.2%, *p*=0.985. Kaplan-Meier survival analysis showed a significant survival benefit in EDA-/- mice over WTs. Most deaths occurred after day 6 and were not caused by cardiac ruptures (only 2 ruptures in the WT and 1 rupture in EDA-/- mice were observed during 28 days follow-up). In line with increased mortality, WT mice had greater LV dimensions and reduced systolic performance compared to EDA-/- mice. These significant differences were already present 7 days after infarction, and continued to deteriorate till 28 days post-MI. EDA-/- mice were relatively protected against remodeling and exhibited better systolic function after MI. The protective effect seen in EDA-/- was not attributable to changes in the extent of viable tissue, because infarct size did not differ between WT and EDA-/- mice 28 days post-MI (33.7±2.3% vs. 34.3±3.5%, respectively; *p*=0.818). However, wall thickness of the infarct area did not decline in EDA-/- mice as much as in WTs. At day 28 post-infarction, the entire infarct area was replaced by a dense collagen network in both groups. At day 7, however, there was reduced granulation of the infarct as shown by delayed degradation of acellular matrix in EDA-/- mice.

### Lack of EDA decreases endogenous MMP-2, -9 and elastase activities

Enhanced MMP2 and -9 activities are detrimental for cardiac performance and geometry during the post-infarct healing process. We performed zymography to study whether the protection against adverse remodeling seen in EDA-/- mice is also attributable to changes in proteinase activity. In line with the reduced matrix degradation in the knock-out animals at day 7 observed in histology, both active forms of MMP-2 and -9 in infarct areas were reduced in EDA-/- mice, 7 days post-infarction. Analysis of the remote area after 7 days infarction was not possible due to very low signal intensity. Finally, we studied whether elastase activity was also affected by the absence of EDA. In line with reduced MMP2 and -9 activity, elastase activity was also reduced in EDA-/- mice 7 days after infarction.

### EDA-/- mice exhibit less post-infarct fibrosis

Collagen deposition occurs in the infarct area upon degradation of the matrix and in the remote myocardium upon changes in wall stress. Myofibroblasts are the primary source of *de novo* collagen synthesis. In our study, collagen deposition in both the infarct and remote area was similar between the groups at day 7 post-MI. After 28 days, collagen content was again similar in the infarct area, suggesting that scar formation is not negatively affected in EDA-/- mice. However, the remote myocardium now contained less collagen fibers in EDA-/- mice compared to WT animals. These findings were supported at the mRNA level. Both procollagen-1 and -3 are reduced in the remote myocardium of EDA-/- mice. Within the infarct, collagen synthesis in EDA-/- mice was again comparable to WT animals. There was no difference in lysyl-oxidase and TIMP-2 production between the groups, suggesting no differences in collagen cross-linking and protease inhibition, respectively. The reduced fibrosis in the remote myocardium at 28 days post-MI is preceded by a significantly decreased myofibroblast transdifferentiation in EDA-/- mice, in both remote and infarct areas 7 days after infarction. Periostin is described as a maturation factor of cardiac fibroblasts. In our study, periostin-positive area was reduced as well in EDA-/- mice compared to WT animals. To study whether WT and EDA-/- myofibroblasts differed in their matrix synthesis activity and MMP expression profile, we cultured post-infarct myofibroblasts and stained for myofibroblast markers and pro-collagen-III. *In vitro,* there were no differences between the two genotypes. In addition, zymography was done using the supernatants of the cells and showed also no differences in MMP2 and -9 activity.

### Lack of EDA results in enhanced inotropy and lusitropy

Altered fibrotic processes in EDA-/- mice indicate that diastolic function could be affected as well. Contractility, as indicated by dP/dTmax, was much higher in EDA-/- mice after 28 days infarction. This confirmed our previous MRI findings, that EDA-/- mice exhibit enhanced systolic performance. Increase of LVEDP and tau is detrimental for heart function and is caused by increase of EDV and/or fibrosis; one of the hallmarks of heart failure. Compared to WT animals, diastolic performance was also significantly enhanced in EDA-/- mice after 28 days infarction. Both parameters were significantly lower in EDA-/- mice compared to WT animals, providing evidence that the improved survival in EDA-/- mice is a consequence of both systolic and diastolic functional improvements.

### EDA regulates post-MI inflammation

Considered as a ligand for TLR2 and 4, lack of EDA should result in a decreased inflammatory status. Neutrophils are the first leukocyte subset migrating upon tissue injury and are known to be associated with the extent of damage. Neutrophil count in the infarct area was not different between the groups. Hereafter, macrophages clear cell debris (e.g. necrotic neutrophils and cardiomyocytes) and, more importantly, initiate the remodeling process after infarction. In our study, the number of macrophages was highly reduced in EDA-/- mice 7 days post-infarction. There were no cells detectable after 28 days infarction in both groups. In concordance with the reduced macrophage influx, levels of TNFα, RANTES, GM-CSF (responsible for recruitment, differentiation and maturation of macrophages) and IL-10 were highly reduced in EDA-/- mice, 7 days after infarction. In contrast, MCP-1 levels were increased in EDA-/- mice compared to WT animals at protein and mRNA level.

### Parenchymal EDA mediates post-infarct survival and maladaptive remodeling

We generated chimeric mice to differentiate between the contribution of the blood and parenchymal compartments to the observed effects after MI. Interestingly, WT/EDA KO BM had similar survival rates and cardiac performance compared to WT/WT BM animals. In contrast, EDA KO/WT BM were similar to EDA KO/EDA KO BM animals and showed higher survival rates and exhibited less adverse remodeling after MI, compared to WT/EDA KO BM. These data indicate that post-infarct parenchymal EDA expression drives maladaptive remodeling. From a danger model perspective, we may postulate that EDA expression as a danger signal can have profound effects on circulating cells which are responsible for post-infarct repair responses.

### EDA mediates both integrin-α4 and Toll-like receptor signaling in circulating monocytes

EDA is a known ligand for integrin-α4β1 (VLA-4) and TLR2 and 4. Since parenchymal EDA mediates adverse remodeling, we hypothesized that EDA from the heart may serve as an endogenous activator of circulating cells after infarction. EDA-/- mice showed a significant reduction in peripheral monocytes 3 days after infarction, whereas after 7 days the numbers were similar between the groups. TLR2 expression on monocytes was significantly altered in the absence of EDA, while TLR4 did not show any difference in expression levels after MI between the groups. Integrin-α4 (CD49d) expression was also significantly reduced on monocytes of EDA-/- mice after infarction. In addition, there was a subgroup of monocytes that showed a significant higher expression level of CD49d. EDA-/- mice showed again a reduced CD49d expression in this subgroup, 7 days post-infarction.

### Example 2

### Methods

### Animals and experimental design

Male Balb/C wild-type mice (10-12 weeks, 25-30 g) received standard diet and water *ad libitum.* Myocardial infarction was induced by left coronary artery ligation, just below the left atrial appendage. All animal experiments were performed in accordance with the national guidelines on animal care and with prior approval by the Animal Experimentation Committee of Utrecht University.

Mouse monoclonal antibodies of IgG1 and IgG2a subclass (Peters JH et al. Blood. 1990. Vol. 75:1801-1808) were tested, recognizing the following peptide (antigen): TYSSPEDGIHELFPAPDGEEDTAELQG, corresponding to amino acids 36 to 60 of the EDA domain of human fibronectin-EDA.

Balb/C mice underwent left coronary artery ligation or sham operation and cardiac function and geometry assessment at baseline, 7 and 28 days after MI using high-resolution 9.4T mouse cardiac magnetic resonance imaging. Mice were given intravenously 250 microliters (µl) of saline (control) or antibody solution via the tail vein. The animals were randomized to receive saline, IgG1 (20 mg/kg) or IgG2a (20 mg/kg) monoclonal antibody 1 day after infarction. The bolus injections were repeated at day 3 and day 5 post-infarction. The 2^{nd} and 3^{rd} antibody injections were given at a dosage of 10 mg/kg.

### Myocardial infarction in vivo

Mice were anesthetized with a mixture of Fentanyl (Jansen-Cilag) 0.05 mg/kg, Dormicum (Roche) 5 mg/kg and medetomidine 0.5 mg/kg through an intraperitoneal injection. Core body temperature was maintained around 37°C during surgery by continuous monitoring with a rectal thermometer and automatic heating blanket. Mice were intubated and ventilated (Harvard Apparatus Inc.) with 100% oxygen. The left coronary artery (LCA) was permanently ligated using an 8-0 vicryl suture. Ischemia was confirmed by bleaching of the myocardium and ventricular tachyarrhythmia. In sham operated animals the suture was placed beneath the LCA without ligating. The chest wall was closed and the animals received subcutaneously Antisedan (Pfizer) 2.5 mg/kg, Anexate (Roche) 0.5 mg/kg and Temgesic (Schering-Plough) 0.1 mg/kg.

### Magnetic resonance imaging

Heart function and geometry assessment was done by a technician blinded to treatment group. The mice underwent serial assessment of cardiac dimensions and function by high resolution magnetic resonance imaging (MRI, 9.4 T, Bruker, Rheinstetten, Germany) under isoflurane anesthesia before, 7 and 28 days after MI. Long axis and short axis images with 1.0 mm interval between the slices were obtained and used to compute end-diastolic volume (EDV, largest volume) and end-systolic volume (ESV, smallest volume). Systolic wall thickening (SWT) was assessed from both the septum (remote myocardium) and free wall (infarct area). All MRI data were analyzed using Qmass digital imaging software (Medis, Leiden, The Netherlands).

### Results

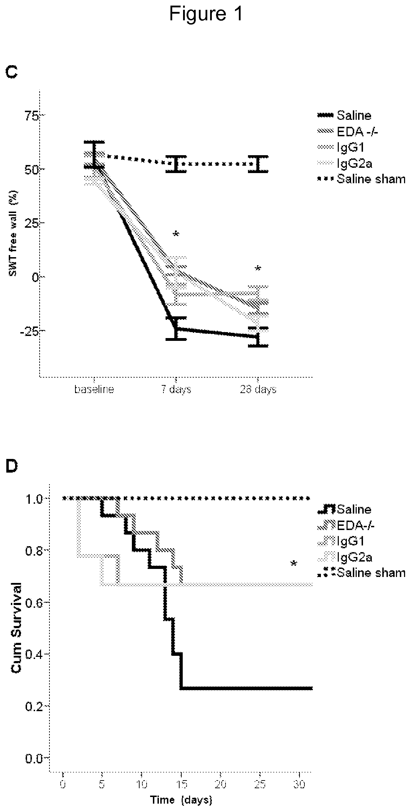

There were no differences between the different groups of mice at baseline (t=0). Mice treated with either IgG1 or IgG2a developed decreased ventricular dilatation compared to saline treated animals. Compared to saline treatment, end-diastolic and end-systolic volume (EDV, ESV) was lower in IgG1 and IgG2a treated animals, indicating decreased ventricular dilatation upon treatment with anti-fibronectin-EDA antibodies (Fig. 1A and B). In addition, anti-fibronectin-EDA treated animals exhibited reduced ventricular bulging as shown by higher SWT index (Fig. 1C). More importantly, the preserved cardiac function and geometry translated into improved survival in anti-fibronectin-EDA treated mice. Compared to saline treated animals, IgG1 and IgG2a treated animals exhibited 50% reduction in mortality during 28 days follow-up after MI (Fig. 1D). Interestingly, the therapeutic effect of both anti-fibronectin-EDA antibodies is comparable to the phenotype of EDA^{-/-} mice after MI (Fig. 1A-D)

In conclusion, IgG1 and IgG2a antibodies against the human fibronectin-EDA segment preserve left ventricular geometry and function and improve survival after MI. The therapeutic effect seen in IgG1 and IgG2a treated mice is comparable to the protective effect of fibronectin-EDA deficiency in mice suggesting a high specificity and safety of IgG1 and IgG2a antibodies against human fibronectin-EDA.

### REFERENCES

1) AHA Heart Disease & Stroke Statistics 2009, Dallas, Texas
2) European Cardiovascular Disease Statistics 2008, Oxford
3) Takii T et al. Increasing Trend of the Incidence of Acute Myocardial Infarction Over 30 Years in Japan: Lessons From the MIYAGI-AMI Registry Study. Circulation. 2009;120:S430
4) ESC World Congress of Cardiology 2006, Barcelona
5) Stakeholders Opinions: Heart Failure 2008, Datamonitor Healthcare
6) Roger VL et al. Trends in heart failure incidence and survival in a community-based population. JAMA. 2004;292:344-350
7) Juenger J *et al.* Health related quality of life in patients with congestive heart failure: comparison with other chronic diseases
8) Frangogiannis NG. The immune system and cardiac repair. Pharmacol Res. 2008;58:88-111

## Claims

1. An antibody specifically binding to the EDA domain of fibronectin-EDA for use in treating, preventing, or preventing progression of, a condition relating to or resulting from myocardial infarction and/or adverse remodeling, wherein said condition is selected from the group consisting of cardiac dilatation, adverse ventricular remodeling, heart failure, remote myocardial fibrosis, aneurysm or rupture of the ventricle, mitral regurgitation, and arrhythmias and wherein said antibody specifically binds to the amino acid sequence TYSSPEDGIHELFPAPDGEEDTAELQG (SEQ ID NO:2).

2. An antibody for use according to claim 1, for improving cardiac function.

3. An antibody for use according to any one of the preceding claims which is a monoclonal antibody.

4. An antibody for use according to claim 3, which is a human antibody.

5. An antibody for use according to claim 3, which is a humanized antibody.

6. An antibody of use according to claim 5, which is a humanized mouse antibody.

## Patentansprüche

1. Antikörper, spezifisch bindend an die EDA-Domäne von Fibronectin-EDA zur Verwendung in der Behandlung, Verhinderung oder Verhinderung des Fortschreitens eines Zustands, im Zusammenhang mit oder resultierend aus Myokardinfektion und/oder ungünstiger Remodellierung, wobei der Zustand ausgewählt ist aus der Gruppe, bestehend aus Herzdilatation, ungünstiger ventikulärer Remodellierung), Herzversagen, Fernmyokardfibrose, Aneurysma oder Riss des Ventrikels, Mitralinsuffizienz und Arrhythmia, und wobei der Antikörper spezifisch an die Aminosäuresequenz TYSSPEDGIHELFPAPDGEEDTAELQG (SEQ ID NR.: 2) bindet.

2. Antikörper zur Verwendung nach Anspruch 1 zur Verbesserung der Herzfunktion.

3. Antikörper zur Verwendung nach einem der vorhergehenden Ansprüche, welcher ein monoklonaler Antikörper ist.

4. Antikörper zur Verwendung nach Anspruch 3, welcher ein humaner Antikörper ist.

5. Antikörper zur Verwendung nach Anspruch 3, welcher ein humanisierter Antikörper ist.

6. Antikörper zur Verwendung nach Anspruch 5, welcher ein humanisierter Maus-Antikörper ist.

## Revendications

1. Anticorps qui se lie spécifiquement au domaine EDA de la fibronectine-EDA destiné à être utilisé dans le traitement, la prévention ou la prévention de la progression de, une affection liée à ou résultant d'un infarctus du myocarde et/ou d'un remodelage défavorable, où ladite affection est choisie dans le groupe consistant en la dilatation cardiaque, le remodelage ventriculaire défavorable, l'insuffisance cardiaque, la fibrose myocardique distante, l'anévrisme ou la rupture du ventricule, la régurgitation mitrale et les arythmies et où ledit anticorps se lie spécifiquement à la séquence d'acides aminés TYSSPEDGIHELFPAPDGEEDTAELQG (SEQ ID NO : 2).

2. Anticorps destiné à être utilisé selon la revendication 1, pour améliorer la fonction cardiaque.

3. Anticorps destiné à être utilisé selon l'une quelconque des revendications précédentes qui est un anticorps monoclonal.

4. Anticorps destiné à être utilisé selon la revendication 3, qui est un anticorps humain.

5. Anticorps destiné à être utilisé selon la revendication 3, qui est un anticorps humanisé.

6. Anticorps destiné à être utilisé selon la revendication 5, qui est un anticorps de souris humanisé.
